# EUROPEAN PATENT SPECIFICATION

(11) **EP 3 511 310 B1**
(45) Date of publication and mention of the grant of the patent: **22.03.2023**
(21) Application number: 16915626.2
(22) Date of filing: 08.09.2016
(51) Int. Cl.: B01D 53/78, B01D 53/14, B01D 53/18, B01D 53/52, C12M 1/107, C12M 1/00

(54) **DEVICE AND METHOD FOR INCREASING THE CONTENT OF METHANE IN A CURRENT OF BIOGAS BY MEANS OF A LOW-PRESSURE AIRLIFT SYSTEM**
VORRICHTUNG UND VERFAHREN ZUR ERHÖHUNG DES GEHALTS VON METHAN IN EINEM BIOGASSTROM DURCH EIN NIEDERDRUCK-LUFTKORRIDORSYSTEM
APPAREIL ET PROCÉDÉ PERMETTANT D'ACCROÎTRE LA TENEUR EN MÉTHANE D'UN COURANT DE BIOGAZ, AU MOYEN D'UN SYSTÈME D'EXTRACTION À L'AIR À BASSE PRESSION

(43) Date of publication of application: 17.07.2019
(73) Proprietor: Inergyclean Technology, S.L., 04131 Almeria (ES)
(72) Inventor: IGLESIAS HERNANDEZ, David, 04131 Almería (ES); ROMAN RANCHAL, Fernando, 04131 Almería (ES); LLAMAS MOYA, Bernardo, 04131 Almería (ES); POZO DENGRA, Joaquín, 04131 Almería (ES)
(74) Representative: González Peces, Gustavo Adolfo
(86) International application number: PCT/ES2016/070632
(87) International publication number: WO 2018/046778

(56) References cited:
- EP-A1- 0 180 670
- EP-A1- 0 252 169
- EP-A1- 3 061 515
- WO-A1-2017/040268
- FR-A1- 2 991 193
- US-A1- 2010 107 872

## Description

### TECHNICAL FIELD OF THE INVENTION

The present invention lies in the field of biomethane production from biogas.

Specifically, the invention relates to a device and method for increasing the methane (CH4) content in a biogas stream using an airlift system, based on washing with water at a low pressure, that is, between 1 and 1.5 atm.

The invention applies to all the biogas produced by anaerobic digestion in organic matter degradation, such as treatment processes of purines, purifier sludge or the organic fraction or solid urban waste.

The invention allows purifying the biogas, obtaining biomethane with concentrations of carbon dioxide (CO2) under 3% and hydrosulfuric acid (H2S) under 150 ppm (0.015%). In other words, most of the carbon dioxide (CO2) and hydrosulfuric acid (H2S) contained in the biogas is eliminated, producing a gaseous stream with a high concentration of methane (CH4) suitable for commercial use.

### BACKGROUND OF THE INVENTION

The anaerobic digestion stage in waste (solid and/or liquid) treatment processes allows stabilizing said waste by transforming organic matter into biogas, the resulting digestate having a potential use as organic fertilizer in the agroindustrial sector, or being dried for its final disposal in dumps or incineration. The biogas produced is a mixture of compounds, mainly methane (CH4) and carbon dioxide (CO2); it is possible to find in the biogas other compounds not intended for use as a fuel, such as water, hydrosulfuric acid (H2S), oxygen, ammonia (NH3), siloxanes, and solid particles, which must be eliminated from the biogas before it is used as a fuel.

Known biogas treatment processes can be differentiated into two stages: refining and purification. The refining stage is meant to eliminate the main undesired component, that is, carbon dioxide (CO2), and the purification stage eliminates the remaining compounds, that is, hydrosulfuric acid (H2S), water,etc.

One of the most common techniques used for refining biogas is absorption by washing with pressurized water, generally between 4 and 6 bar. This technique uses the ability of water to absorb carbon dioxide (CO2) compared to other less soluble gases, such as methane (CH4), where this difference in solubility is greater the higher the pressure in the system. Specifically, the solubility of carbon dioxide (CO2) in water is 26 times greater than that of methane (CH4).

A basic system for this technique comprises an absorption column and a desorption column, when a water regeneration process is desired, as well as a flash column to recover the methane (CH4) absorbed by the water. The absorption column is generally a structured filling column, meant to increase contact between the two fluids, namely the biogas and the water. In this absorption process with pressurized water the absorption of other compounds also occurs, such as hydrosulfuric acid (H2S) and ammonia (NH3), which are also gases more soluble than methane(CH4). This allows using this technology with a biogas having an initial concentration of hydro sulfuric acid (H2S) of 300-2500ppm that is, without requiring a pre-treatment of the biogas to reduce the concentration of hydrosulfuric acid (H2S) at the inlet of the refining stage.

However, these biogas refining techniques using absorption by washing with pressurized water have serious drawbacks related to the high initial investments required, and during operation the risks involved in handling explosive gases at high pressure, the high water consumption, and the high energy consumption related to operating the pumps and compressors for operating the installation.

For example, EP0252169 shows a process for the production of biogas from, in particular, agricultural waste, wherein the biogas produced is subjected to desulphurization by introducing it into water by means of one or more immersion tubes, which is constantly renewed by constant fresh water supply and simultaneous discharge is carried out. The immersion tubes are arranged in a U-shaped water tank with a fresh water inlet and an overflow.

For example, WO2017/040268 shows a system and method for performing a water wash process on a biogas supply. Multiple risers are provided to perform different steps in the water wash process, wherein, carbon dioxide and other soluble constituents are absorbed from the biogas stream. The absorption is performed by utilizing a series of absorption risers configured to be installed below grade. The inlets for receiving a biogas stream and a water stream are located at a top end of the riser. The outlets for removing the purified gas stream and mixed water stream are also located at the top end of the riser. The riser may then be located substantially below grade such that the top end is accessible at or just above the ground level.

Another example is shown in EP3061515, which is a system for cleaning and upgrading biogas having hydrogen sulphide concentration by means of physical water absorption, the system comprising a first pipe line that provides wash water to the system; a second pipe line that provides the biogas to the system; a counter-current absorption bubble column for simultaneous removal of hydrogen sulphide and carbon dioxide by physical absorption, the bubble column comprising: a first area wherein the wash water enters downwards through the first pipe line by gravity and the biogas is injected through the second pipe line in counter-current conditions by means of one or more fine bubble diffusers located below the first pipe line level; a second area located below and adjacent to the first area to prevent bubbles from leaving with the outlet water; and a third area located all along the column having a vertical baffle to separate the used wash water flow from the first and the second areas, and a wash water outlet at the top thereof.

For this reason it is necessary to design, in a simple and economic manner, a solution that increases the methane content in a biogas stream by washing with water at low pressure, that is, from 1 to 1.5 atm, solving the aforementioned drawbacks in the prior art.

### DESCRIPTION OF THE INVENTION

The present invention is established and characterized in the independent claims, while the dependent claims describe additional characteristics thereof. The present invention relates to a device and a method for increasing the methane content in a biogas stream by washing with water at low pressure. The technical problem to be solved is preventing the risks involved in handling explosive gases at a high pressure, as well as reducing the power consumption and cost of the device itself. The invention manages to solve the technical problem addressed effectively, as the design of the carbon dioxide (CO2) and hydrosulfuric (H2S) acid absorption column according to claim 1 allows obtaining a biogas with an increased methane content (CH4) without requiring the handling of explosive gases at high pressure. That is, the biogas is purified at low pressure, between 1 and 1.5 atm, preferably at 1 atm, without involving compression of the biogas at high pressure nor subjecting same to great temperature changes. In addition, no corrosive, toxic or polluting reagents are used. It is thereby possible to remove from the biogas over 95% of the carbon dioxide (CO2) and hydrosulfuric acid (H2S).

In addition, it also constitutes a solution with low investment costs, that does not require a high water consumption and represents a significant reduction in the power consumption of the biogas refining stage. It is a robust solution, with an operation that does not have complex mechanical parts that may be damaged nor sophisticated control mechanisms. This solution operates according to simple and universal principles of physics and chemistry.

### BRIEF DESCRIPTION OF THE FIGURES

This specification is supplemented with a drawing illustrating the preferred embodiment, which is never intended to limit the invention.

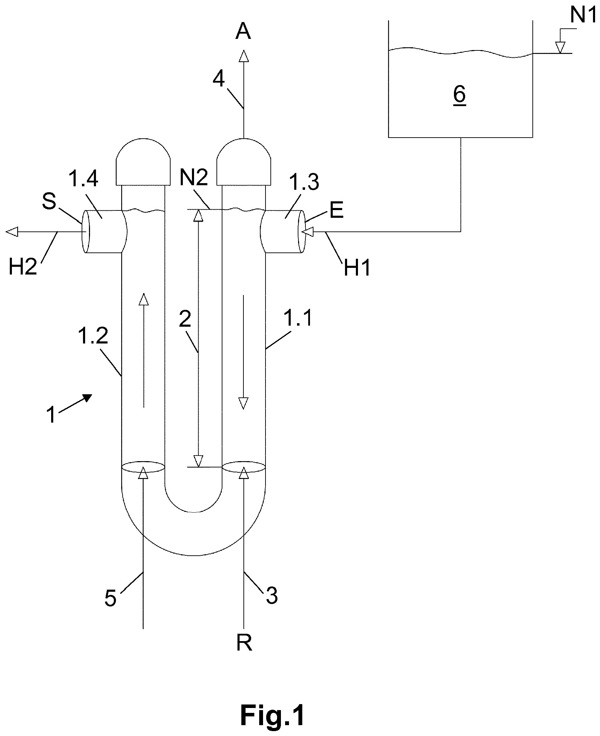

Figure 1 shows a schematic view of a first example of an embodiment of the device for increasing the methane content in a biogas stream using an airlift system body.

### DETAILED DESCRIPTION OF THE INVENTION

The present invention relates to a device for increasing the methane (CH4) content in a biogas stream, the latter also containing carbon dioxide (CO2) and hydrosulfuric acid (H2S). As shown in figure 1, the device comprises at least one airlift system body (1) which in turn comprises a downward duct (1.1) and an upward duct (1.2) through which travels a flow of water (Hl) at a pressure of 1 to 1.5 atm, preferably at atmospheric pressure.

The airlift system body (1) can be understood as the containing medium in which a liquid (in this case water) flows propelled by an airlift system, and where the biogas is injected for the absorption and refining process of said biogas.

The airlift system body (1) is preferably formed by a curved tubular element with a U shape having a water inlet (1.3) and a water outlet (1.4) arranged at the upper ends of the corresponding upward duct (1.2) and downward duct (1.1). However, the airlift system body (1) can have any other shape, provided said upward and downward ducts (1.1, 1.2) are defined therein.

The water flow (Hl), preferably of anoxic water, that is, water without oxygen, can be provided by a tank (6) with a liquid level (Nl) located above a second liquid level (N2) of the water inlet (1.3) of the airlift system body (1). This ensures that between the water inlet (1.3) and the water outlet (1.4), the upward and downward ducts (1.1, 1.2) are completely covered by water. The water will thus travel under the action of gravity through the airlift system body (1) without the need for pumping means.

The device also comprises propulsion means (5) for the water flow arranged in the upward duct (1.2) of the airlift system body (1). The propulsion means (5) change the pressure in the water flow, increasing the flow rate of water through the airlift system body (1) toward the water outlet (1.4), where water with an increased oxygen content (H2) arrives.

In addition the water (Hl) to be transferred can be waste water or water in anoxic conditions obtained from a previous biological treatment under anaerobic conditions or chemical treatment with bisulphite. If waste water is used, the integration of the device in a waste water purification line will also allow regenerating water with increased oxygen content (H2) resulting from the operation of the device.

Additionally, the biogas stream to be refined (R) can come from any biogas source due to anaerobic digestion in organic matter breakdown processes, such as sludge, solid urban waste, etc. The device also comprises an absorption column (2), included in the airlift system body (1), where the water flow absorbs carbon dioxide (CO2) and hydrosufuric acid (H2S) contained in the biogas, the latter being introduced in the airlift system body (1) by injection means (3) arranged in the lower part of the absorption column (2) provided. That is, contact of the biogas with the water flow (H1) along the absorption column (2) causes the transfer to the water of carbon dioxide (CO2) and hydrosulfuric acid (H2S) content in the biogas, while the biogas with increasing methane content (CH4) rises up the absorption column (2).

In addition, the device comprises recovery means (4) for the biogas with increased methane (CH4) content, located at the upper part of the absorption column (2). Thus, the biogas with increased methane (CH4) content that rises up the absorption column (2) and passes the second liquid level (N2) is caught by the recovery means (4) and led to the biogas purification stage (A) or to a new step of the refining stage.

In the invention, represented in figure 1, the absorption column (2) is placed in the downward duct (1.1) of the airlift system body (1), and the injection means (3) introduce the biogas against the flow of the water. In addition, the propulsion means (5) introduce air in a co-flow with the water flow.

For example, in the following conditions:
- absorption column (2) with a height of approximately 2 meters and a diameter of approximately 5 cm,
- pressure of the water and biogas stream at atmospheric pressure,
- ratio of water to biogas between approximately 1 and approximately 3 m³/m³, preferably 1.6 m³/m³,
- stream of biogas to refine (R) with approximately 60% methane (CH4), approximately 39% carbon dioxide (CO2) and approximately 1% hydrosulfuric acid (H2S).

At the outlet of the device a stream of biogas is obtained with a content of methane (CH4) with approximately 95% methane (CH4), 2% carbon dioxide (CO2) and approximately 0.01% hydrosulfuric 10 acid (H2S).

As can be seen at the outlet of the device a gaseous stream is obtained with a high concentration of methane (CH4), suitable for being led to the purification stage (A) of the biogas in order to obtain biogas for commercial uses, for injection into the natural gas grid, or for use as a biofuel.

The present invention also relates to a method for increasing the methane (CH4) content in a biogas stream, which can be used with any embodiment of the device described above.

The method starts with the transfer of a water flow (Hl), at a pressure between 1 and 1. 5 atm, preferably at atmospheric pressure, through the airlift system body (1) provided in the device.

Next the stream of biogas to be refined (R) is injected in the lower part of an absorption column (2) provided in the airlift system body (1).

In the absorption column (2), carbon dioxide and hydrosulfuric acid (H2S) content is transferred from the biogas to the water flow, while the biogas with an increased methane (CH4) content rises through the absorption column (2) until passing through the surface of the water (N2). The biogas with increased methane (CH4) content accumulated in the absorption column (2) is then recovered. As a result of this method a gaseous stream is obtained with a high methane (CH4) content that can be led to a biogas purification stage (A) in which the rest of the undesired minority components can be eliminated, such as siloxanes, carbon monoxide (CO), nitrogen oxides (NOx) etc. in order to obtain biomethane for commercial use.

## Claims

1. Device for increasing the methane (CH4) content in a biogas stream that also contains carbon dioxide (CO2) and hydrosulfuric acid (H2S), comprising:
an airlift system body (1) comprising a first duct (1.1) and a second duct (1.2), adapted to pass a flow of water at a pressure from 1 to 1.5 atm downward through the first duct (1.1), and upward through the second duct (1.2), an absorption column (2) provided in the first duct (1.1), injection means (3) of the biogas stream arranged in the first duct (1.1) of the airlift system body (1) at the lower part of the absorption column for introducing a biogas against the flow of water, where the water flow can absorb carbon dioxide (CO2) and hydrosulfuric acid (H2S) contained in the biogas, recovery means (4) for the biogas with increased methane (CH4) content, arranged at the upper part of the absorption column (2), **characterized in that** it comprises propulsion means (5) for the water flow arranged in the second duct (1.2) of the airlift reactor body (1), the propulsion means (5) are adapted to introduce an air flow in a co-current with the water flow.

2. Device according to claim 1, where the airlift system body (1) is formed by a U-shaped tubular element.

3. Method for increasing the methane (CH4) content in a biogas stream that also contains carbon dioxide (CO2) and hydrosulfuric acid (H2S), comprising the following steps:
a) passing a water flow at a pressure from 1 to 1.5 atm through an airlift system body (1) with a first duct (1.1) and a second duct (1.2), wherein the water flow passes downward through the first duct (1.1) and upward through the second duct (1.2);
b) injecting biogas against the flow of water in the first duct (1.1) of the airlift system body (1) at the lower part of an absorption column (2) located in the first duct (1.1);
c) transferring content of carbon dioxide (CO2) and hydrosulfuric acid (H2S) from the biogas to the water flow, in the absorption column (2); and
d) recovering biogas with increased methane (CH4) content accumulated on the absorption column (2);
**characterized in that** the transfer of the water flow is produced by propulsion means (5) in the second duct (1.2) that introduce air in a co-current with the water flow.

4. Method according to claim 3, where the ratio of transferred water to injected biogas is between approximately 1 m³/m³ and approximately 3 m³/m³.

## Patentansprüche

1. Vorrichtung zur Erhöhung des Methan(CH4)-Gehalts in einem Biogasstrom, der auch Kohlendioxid (CO2) und Schwefelwasserstoffsäure (H2S) enthält, umfassend:
einen Lufthebesystemkörper (1), der eine erste Leitung (1.1) und eine zweite Leitung (1.2) umfasst, der geeignet ist, einen Wasserstrom mit einem Druck von 1 bis 1,5 atm durch die erste Leitung (1.1) nach unten und durch die zweite Leitung (1.2) nach oben zu leiten, eine Absorptionssäule (2), die in der ersten Leitung (1.1) vorgesehen ist, Einspritzmittel (3) für den Biogasstrom, die in der ersten Leitung (1.1) des Lufthebesystemkörpers (1) am unteren Teil der Absorptionssäule angeordnet sind, um ein Biogas gegen den Wasserstrom einzuleiten, wobei der Wasserstrom das im Biogas enthaltene Kohlendioxid (CO2) und die Schwefelwasserstoffsäure (H2S) absorbieren kann, am oberen Teil der Absorptionssäule (2) angeordnete Rückgewinnungsmittel (4) für das Biogas mit erhöhtem Methan(CH4)-Gehalt, **dadurch gekennzeichnet, dass** sie Antriebsmittel (5) für den Wasserstrom umfasst, die in der zweiten Leitung (1. 2) des Lufthebesystemkörpers (1) angeordnet sind, wobei die Antriebsmittel (5) geeignet sind, einen Luftstrom in einem Gleichstrom mit dem Wasserstrom einzuleiten.

2. Vorrichtung gemäß Anspruch 1, wobei der Lufthebesystemkörper (1) durch ein U-förmiges rohrförmiges Element gebildet wird.

3. Verfahren zur Erhöhung des Methan(CH4)-Gehalts in einem Biogasstrom, der auch Kohlendioxid (CO2) und Schwefelwasserstoffsäure (H2S) enthält, umfassend die folgenden Schritte:
a) Leiten eines Wasserstroms mit einem Druck von 1 bis 1,5 atm durch einen Lufthebesystemkörper (1) mit einer ersten Leitung (1.1) und einer zweiten Leitung (1.2), wobei der Wasserstrom durch die erste Leitung (1.1) nach unten und durch die zweite Leitung (1.2) nach oben fließt;
b) Einspritzen von Biogas gegen den Wasserstrom in der ersten Leitung (1.1) des Lufthebesystemkörpers (1) am unteren Teil einer Absorptionssäule (2), die sich in der ersten Leitung (1.1) befindet;
c) Übertragen von Gehalt an Kohlendioxid (CO2) und Schwefelwasserstoffsäure (H2S) aus dem Biogas auf den Wasserstrom in der Absorptionssäule (2); und
d) Rückgewinnung von Biogas mit erhöhtem Methan(CH4)-Gehalt, das sich in der Absorptionskolonne (2) angesammelt hat;
**dadurch gekennzeichnet, dass** die Übertragung des Wasserstroms durch Antriebsmittel (5) in der zweiten Leitung (1.2) erzeugt wird, die Luft in einem Gleichstrom mit dem Wasserstrom einleiten.

4. Verfahren gemäß Anspruch 3, wobei das Verhältnis von übertragenem Wasser zu eingespritztem Biogas zwischen etwa 1 m³/m³ und etwa 3 m³/m³ liegt.

## Revendications

1. Appareil permettant d'accroître la teneur en méthane (CH4) d'un flux de biogaz contenant également du dioxyde de carbone (CO2) et de l'acide hydrosulfurique (H2S), comprenant :
un corps de système d'extraction à l'air (1) comprenant un premier conduit (1.1) et un second conduit (1.2), apte à laisser passer un écoulement d'eau à une pression de 1 à 1,5 atm vers le bas à travers le premier conduit (1.1), et vers le haut à travers le second conduit (1.2), une colonne d'absorption (2) prévue dans le premier conduit (1.1), des moyens d'injection (3) du flux de biogaz agencés dans le premier conduit (1.1) du corps de système d'extraction à l'air (1) au niveau de la partie inférieure de la colonne d'absorption pour introduire un biogaz contre l'écoulement d'eau, dans lequel l'écoulement d'eau peut absorber du dioxyde de carbone (CO2) et de l'acide hydrosulfurique (H2S) contenus dans le biogaz, des moyens de récupération (4) pour le biogaz avec une teneur accrue en méthane (CH4), agencé au niveau de la partie supérieure de la colonne d'absorption (2), **caractérisé en ce qu'**il comprend des moyens de propulsion (5) pour l'écoulement d'eau agencé dans le second conduit (1.2) du corps de système d'extraction à l'air (1), les moyens de propulsion (5) sont aptes à introduire un écoulement d'air dans un courant commun avec l'écoulement d'eau.

2. Appareil selon la revendication 1, dans lequel le corps de système d'extraction à l'air (1) est constitué d'un élément tubulaire en forme de U.

3. Procédé permettant d'accroître la teneur en méthane (CH4) d'un flux de biogaz contenant également du dioxyde de carbone (CO2) et de l'acide hydrosulfurique (H2S), comprenant les étapes suivantes :
a) le passage d'un écoulement d'eau à une pression de 1 à 1,5 atm à travers un corps de système d'extraction à l'air (1) avec un premier conduit (1.1) et un second conduit (1.2), dans lequel l'écoulement d'eau passe vers le bas à travers le premier conduit (1.1) et vers le haut à travers le second conduit (1.2) ;
b) l'injection de biogaz contre l'écoulement d'eau dans le premier conduit (1.1) du corps de système d'extraction à l'air (1) au niveau de la partie inférieure d'une colonne d'absorption (2) située dans le premier conduit (1.1) ;
c) le transfert d'une teneur en dioxyde de carbone (CO2) et en acide hydrosulfurique (H2S) du biogaz à l'écoulement d'eau, dans la colonne d'absorption (2) ; et
d) la récupération de biogaz avec une teneur accrue en méthane (CH4) accumulé sur la colonne d'absorption (2) ;
**caractérisé en ce que** le transfert de l'écoulement d'eau est produit par des moyens de propulsion (5) dans le second conduit (1.2) qui introduisent de l'air dans un courant commun avec l'écoulement d'eau.

4. Procédé selon la revendication 3, dans lequel le rapport de l'eau transférée sur le biogaz injecté est entre environ 1 m³/m³ et environ 3 m³/m³.
